# EUROPEAN PATENT APPLICATION

(11) **EP 0 839 546 A2**
(43) Date of publication of application: **06.05.1998**
(21) Application number: 97307697.9
(22) Date of filing: 30.09.1997
(51) Int. Cl.: A61M 16/12

(54) **System for monitoring therapy during calibration**

(30) Priority: 02.10.1996 US 27142 P; 13.12.1996 US 764569
(71) Applicant: OHMEDA INC., Liberty Corner, New Jersey 07938-0804 (US)
(72) Inventor: Bathe, Duncan P., Madison, Wisconsin 53711 (US)
(74) Representative: Bousfield, Roger James

(57) **Abstract**

A system for providing nitric oxide therapy to a patient, the system having a source of NO containing gas, a source of O₂ containing gas, at least one gas monitor and an electrically operated valve for controlling the amount of NO containing gas administered to the patient, a therapy CPU providing an electrical signal for controlling the position of the electrically operated valve and for continuously monitoring the value of the electrical signal provided to the electrically operated valve, the improvement comprising a means to activate a calibration system to effect the calibration of the at least one gas monitor whereby the at least one gas monitor is inactivated from monitoring a gas, the means to activate the calibration system providing a signal to the therapy CPU to cause the CPU to detect the value of the electrical signal then being provided to the electrically operated valve by the therapy CPU, means to determine limits of change of the electrical signal with respect to the detected signal value and means to continuously monitor the electrical signal to the electrically operated valve and to provide an alarm signal wherever the monitored signal exceeds the determined limit.

## Description

This invention relates to a system for the administration of inhaled nitric oxide (NO) to a patient for therapeutic effects, and, more particularly, to a system that provides a means of monitoring any failure in the therapy system during the calibration of the various monitors that continually monitor the gases involved in such system.

Nitric oxide is used as a method of therapy and is administered to a patient for various therapeutic reasons, among them the treating and preventing of bronchoconstriction or reversible pulmonary vasoconstriction. The therapy is carried out over an extended period of time and may last for periods up to or even exceeding 28-30 days. During such periods it is necessary to recalibrate the various monitors that are continuously monitoring the streams of therapy gases in the system.

In particular, a zero calibration is needed at least once a day and a span calibration should be done about once a month. The zero calibration requires drawing in room air to carry out the calibration and normally may take about five minutes to carry out full calibration. As to the span calibration, the system draws in calibration gases from other sources and may take five or more minutes to calibrate each monitor. Since the span calibration must be made for the NO, O₂ and NO₂ monitors, that calibration may take up to about 15-20 minutes.

During the time periods in which calibration is taking place, the overall NO administration system is devoid of the normal alarms since the normal monitors are off stream and are not active to carry out the monitoring of the various gases. Due to the sensitivity of the NO administration, it is, of course, extremely important to maintain some alarms or monitoring of the system, particularly in that a toxic substance, NO₂, is continuously being generated in the reaction between NO and O₂ and its concentration must be maintained below the point where it could cause harm to the patient.

A typical system for NO therapy is shown and described in US Patent No. 5,558,083 and it will be noted that the monitors in that system provide monitoring of the various gases such as NO, NO₂, O₂ and possibly others. With that system, the delivery of NO is independent of the monitoring of NO and allows for the delivery of NO without monitoring. The principle behind the delivery of NO of that system is the measurement of the patient flow rate and the closed loop control of NO flow rate using a therapy CPU, an NO flow signal, a user inputted NO concentration value and a flow control valve. If a failure occurred, such as a patient flow sensor, under normal operation, the NO monitor would detect the failure by a change in the gas concentration measurement and inform the user by an audible and visual alarm. However, if the NO monitor is being calibrated at that time of the failure and thus not on stream, the above failure would not be detected.

Accordingly, where there are substantial periods of time during calibration when the monitors are basically inoperative, there is a need to provide some alternate system for insuring that the therapy to the patient does not result in increased risk to the patient.

In accordance with the present invention, a system is provided that continuously allows monitoring of the overall NO administration system even when the monitors are being calibrated and therefore not actively monitoring any of the gases to the patient. The system is initiated when the operator enters the calibration procedure and which sends a signal to the therapy CPU informing it of the lack of gas monitoring. That CPU in normal operation continues to receive a signal in the form of a current that also controls the position of the control valve, i.e. the current to the valve is proportional to the flow through the valve and any change in that current indicates a change in the NO flow and, therefore, a change in the therapy to the patient.

Generally that signal is ignored by the therapy CPU and is not used, however, when the CPU becomes aware of the activation of calibration, it monitors and creates a setting for the current draw at the time the calibration procedure is commenced. The therapy CPU thereafter sets up alarm limits around that initial current value, typical would be a plus or minus 60 percent of the initial current value. The CPU thus continues to monitor that current to the control valve and signals an alarm condition if that current increases or decreases to a value outside the alarm limits set by the CPU and thus alarms on any changes of NO flow in excess of the established limits.

Thus, an alarm is activated and the flow of NO to the patient is continually monitored even during the periods that the gas monitors are basically inactive due to their calibration and the system can continue delivering the therapy to the patient with safety.

For a better understanding of the invention, reference will now be made, by way of exemplification only, to the accompanying drawings, in which:
Figure 1 is a block diagram of a nitric oxide administration system usable with the present invention in its normal operation; and
Figure 2 is a block diagram of the system of Figure 1 where the system is in its calibration cycle in accordance with the present invention.

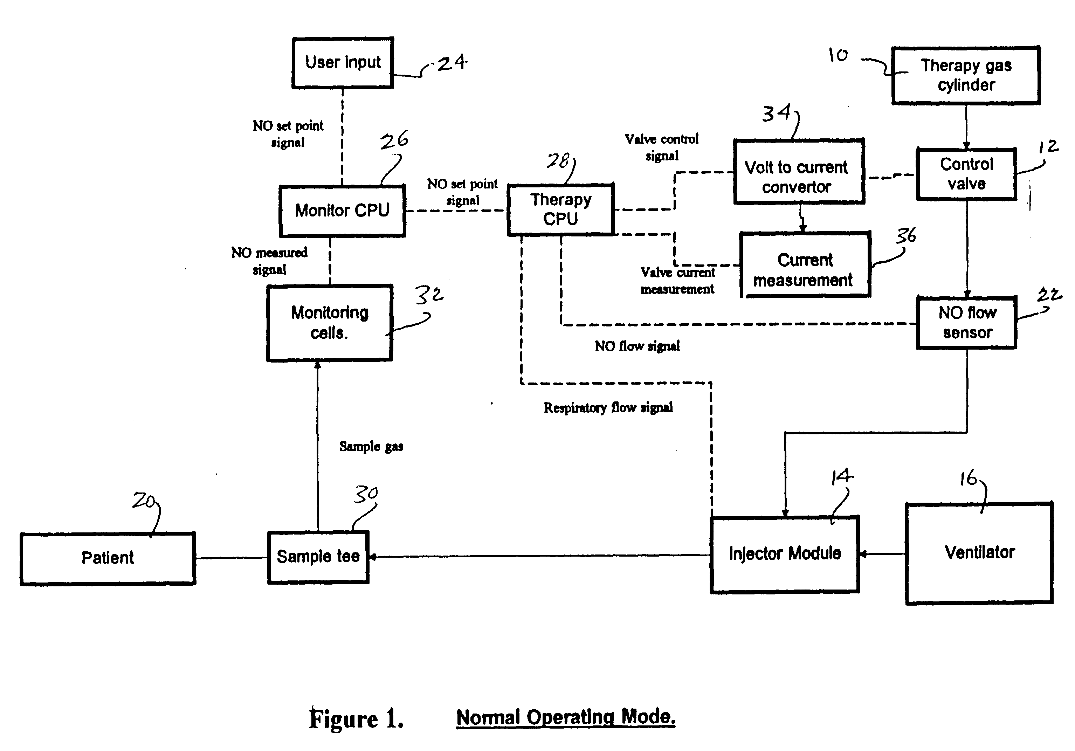

Turning first to Figure 1, there is shown a block diagram of the nitric oxide administration system used with the present invention and which is more fully described in US Patent No. 5,558,083. As shown in Figure 1, however and which is somewhat simplified with respect to the aforementioned US Patent, a supply of nitric oxide is provided in the form of a cylinder 10 of gas. That gas is preferably nitric oxide mixed with nitrogen and is commercially available in a pressurised cylinder or, may be available from a hospital piping system to supply various locations through out the patient care facility such as operating rooms. The supply of NO may, of course, be mixed with nitrogen or other gases to bring the concentration of NO down to a relatively low level.

A proportion gas control valve 12 is positioned with suitable conduit to receive the NO/nitrogen gas from cylinder 10 and a typical suitable proportional control valve 12 is available from Parker Hannifin Corp., Pneutronics Division, Holis, New Hampshire, USA, and which provides electronic control of gases. The flow of NO/nitrogen is thereafter supplied to an injector module 14 where it is mixed with air or other breathable mixture that is supplied from a gas administration device such as ventilator 16. As again will be seen, although a ventilator is shown, the supply of air or other breathable mixture to the patient may be supplied by a manually squeezed bag or other device to breathe the patient.

The injector module mixes the breathable air from ventilator 16 with the supply of NO/nitrogen from the cylinder 10 in the amount desired by the user for ultimate supply to the patient 20. A flow sensor 22 is normally also present in the NO/nitrogen stream to the injector module 14 to monitor the NO flow.

As more detailed in the aforementioned US Patent, the user selects the desired concentration of NO that is suitable for the patient therapy and inputs that selected concentration by an user input 24 into a monitor CPU 26 as a user set point signal. That signal is then transmitted to a therapy CPU 28 and which controls the proportional control valve 12 by sending a signal to that proportional control valve 12 to set the valve to provide the amount of NO/nitrogen to arrive at the desired concentration of NO administered to the patient.

The value of the electrical valve control signal is determined from an input from a flow sensor in the injector module 14 of the flow to the patient and the desired user input set by the user. With those values, the therapy CPU 28 calculates the correct setting of the proportional control valve 12 and sends the appropriate valve control signal to the proportional control valve 12 to achieve the desired user value.

As a further check of the concentration of NO administered to the patient, a sample tee 30 is provided at a point near the patient and a sample of the gas delivered to the patient is extracted and delivered to various monitoring cells 32 where the monitors, generally electrochemical cells, monitor NO, O₂ and NO₂. Those monitored values are provided to the monitor CPU 26 to maintained track of the values administered to the patient and to inform the user that the value of NO is within a close range of the value set by the user.

Taking now, the valve control signal that is provided by the therapy CPU 28 to the proportional control valve 12, various electrical or even light signals may be used, however, in the preferred embodiment, a voltage is outputted by the therapy CPU 28 and which is converted to a proportional current in a voltage to current converter 34 and thus a current indicative of the desired position of proportional control valve 12 is inputted to proportional control valve 12. That current is preferably monitored by a current sensing device 36 and that monitored current again inputted to the therapy CPU 28.

Accordingly, as now can be seen, the user determines the desired therapy concentration of NO to be administered to the patient 20 and inputs that value with the user input 24. The user input 24 establishes a signal indicative of the desired NO concentration to the patient and sends that signal to the monitor CPU 26 and on to the therapy CPU 28. In the therapy CPU, a signal is determined that is to be sent to the proportional control valve 12 based on the user input signal and the flow that is being delivered to the patient through injector module 14 so that the proportional control valve 12 can provide the precise amount of NO in nitrogen to arrive at the desired user value to the patient. The signal to the proportional control valve 12 may be an electrical signal in accordance with the preferred embodiment or may be a electromagnetic signal transmitted by a fibreoptic cable or may even be a mechanical transmission.

In the preferred embodiment, the signal from the therapy CPU 28 is in the form of a voltage that is converted to a current in the voltage to current converter 34 and the current transmitted to the proportional control valve 12 to establish the necessary valve setting. As also noted, the current is monitored by the current sensing device 36, however, during normal operation, that value of current is available to the therapy CPU 28 but is not used for any purpose.

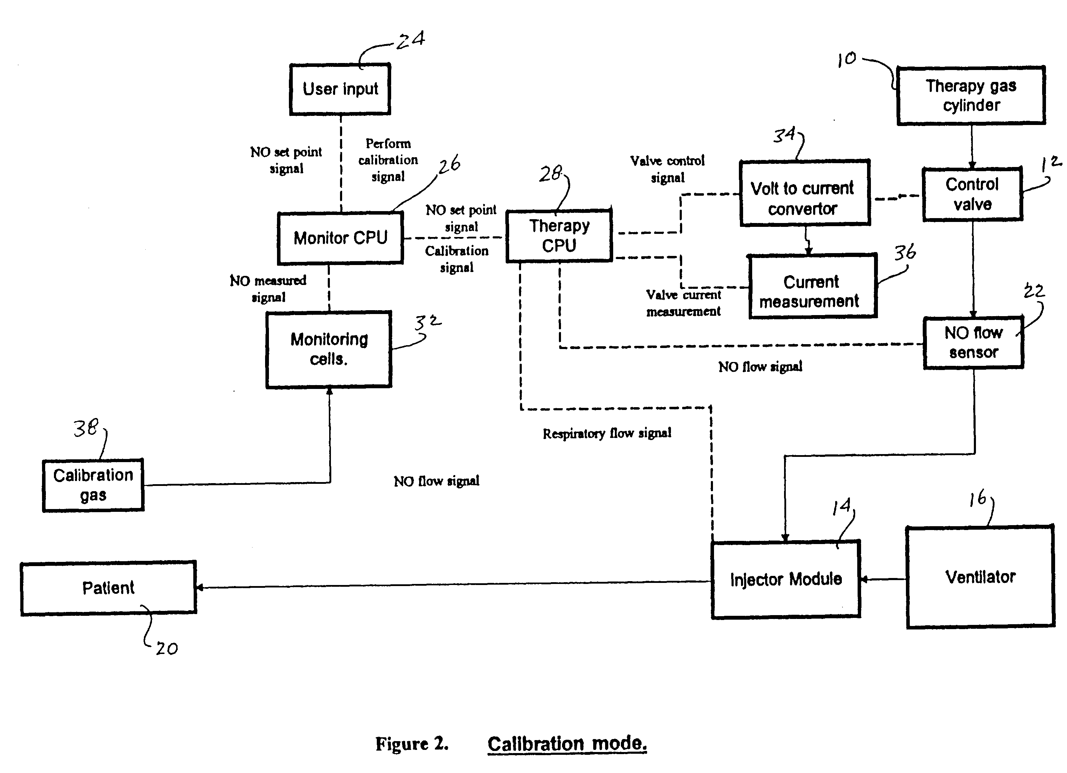

Turning now to Figure 2, there is shown a block diagram of the overall NO administration system when the system is in the calibration mode, that is, the monitoring cells 32 are being calibrated and thus are not available to carry out the continued monitoring of the gases being administered to the patient. In such situation, there is a possibility of a erroneous gas concentration being given to the patient 20 and since one of the gases that is formed with the reaction of O₂ and NO is a toxic gas, NO₂, the continued monitoring of some kind is needed.

As shown in Figure 2, therefore, a calibration gas supply 38 is present and which supplies one or more calibration gases to the monitoring cells 32 to ascertain if the monitoring cells are correctly calibrated. For the O₂ cell, the calibration gas may be air, however for the other cells, a calibration gas or gases are used containing a concentration of NO, and NO₂. In the preferred embodiment the calibration mode is user activated, that is, the user activates the calibration mode by pushing a button or other device on the control panel. Alternatively, of course, the calibration cycle may be timed and a timer continuously tracks the time between calibration and activates the calibration mode at specific intervals.

In either event, when the system goes into calibration mode, a signal is provided to the therapy CPU 28 indicating the initiation of that mode and the therapy CPU 28 immediately determines the magnitude of the signal to it, at that time being transmitted to the proportional control valve controlling the NO/nitrogen gas to the overall system. Thus, the therapy CPU determines and holds that signal value. In the preferred embodiment, that signal is the average current over a ten (10) second period being provided to the proportional control valve 12 to establish the position of that valve. The therapy CPU 28 then establishes a window of values based on the initial value that it has recorded at the time the NO administration system has gone into the calibration mode. As an example, the window may be set at plus or minus 20 percent of the established current average and the therapy CPU 28 sets upper and lower values as alarm limits.

Thus with the alarm limits established, the therapy CPU continues to monitor the current to the proportional control valve 12 throughout the calibration mode and if that current exceed the maximum window value or falls below the minimum window value, an alarm is triggered to alert the user of the condition. That alarm may be audible, visual or both and may also automatically discontinue the administration of NO.

Therefore, as can be seen even though the monitoring cells 32 are being calibrated and thus are no longer actively monitoring the concentration of the gases to the patient 20, the therapy CPU 28 establishes a set point, that of the value to the control signal being sent to the proportional control valve 12, at the time the system is put into calibration mode and the therapy CPU thereafter continues to monitor the signal to the proportional control valve during the calibration mode. If that signal increase above a set cushion above the established value or falls below that cushion , an alarm is triggered to alert the user. Accordingly, even though the normal monitoring cells 32 are inactive, a secondary alarm system is enabled to alert the operator if the proportional control valve 12 is told to change its setting above or below a predetermined amount and thus a monitor is maintained in the NO administration system even when the calibration mode is being activated.

## Claims

1. A system for providing nitric oxide therapy to a patient, the system having a source of NO containing gas, a source of O₂ containing gas, at least one gas monitor and an electrically operated valve for controlling the amount of NO containing gas administered to the patient, a therapy CPU providing an electrical signal for controlling the position of the electrically operated valve and for continuously monitoring the value of the electrical signal provided to the electrically operated valve, the improvement comprising a means to activate a calibration system to effect the calibration of the at least one gas monitor whereby the at least one gas monitor is inactivated from monitoring a gas, the means to activate the calibration system providing a signal to the therapy CPU to cause the CPU to detect the value of the electrical signal then being provided to the electrically operated valve by the therapy CPU, means to determine limits of change of the electrical signal with respect to the detected signal value and means to continuously monitor the electrical signal to the electrically operated valve and to provide an alarm signal wherever the monitored signal exceeds the determined limit.

2. A system according to Claim 1 in which the electrical signal is current.

3. A system according to Claim 1 or Claim 2 in which means to monitor the electrical signal monitors the average current over a predetermined period of time.

4. A system according to any preceding Claim in which the means to determine the limits is a CPU.

5. A system according to any preceding Claim in which the at least one gas monitor monitors NO.

6. A system according to any preceding Claim in which the limits are set as a high limit above the detected electrical signal and a low limit below the detected electrical signal.

7. A method of providing nitric oxide therapy to a patient, comprising the steps of:
providing a source of NO containing gas and a source of O₂ containing gas,
providing at least one gas monitor for monitoring the concentration of a gas being provided to the patient
providing an electrically operated valve for controlling the amount of NO containing gas administered to the patient,
providing a therapy CPU having an electrical signal output
controlling the position of the electrically operated valve by the electrical signal output of the therapy CPU,
continuously monitoring the value of the electrical signal output to the electrically operated valve
calibrating, at selected times, the at least one gas monitor by inactivating the at least one gas monitor and passing a calibration gas therethrough,
detect the value of the electrical signal then being provided to the electrically operated valve by the therapy CPU when the at least one gas monitor is selected for calibration,
determining limits of change of the electrical signal with respect to the detected signal value and continuously monitoring the electrical signal to the electrically operated valve and
activating an alarm wherever the detected signal exceeds the limits of change determined in the previous step.

8. A method according to Claim 7 in which the step of determining limits of change determines both a maximum positive change and a negative signal change with respect to the detected signal value.

9. A method according to Claim 7 or Claim 8 in which the step of continuously monitoring the electrical signal value monitors current.

10. A method according to Claim 9 in which the step of continuously monitoring the electrical signal value monitors the average current over a predetermined period of time.
